# EUROPEAN PATENT SPECIFICATION

(11) **EP 4 397 953 B1**
(45) Date of publication and mention of the grant of the patent: **11.03.2026**
(21) Application number: 23843350.2
(22) Date of filing: 19.07.2023
(51) Int. Cl.: G01N 1/22, G01N 33/00, H01M 10/04

(54) **GAS COLLECTION DEVICE**
GASSAMMELVORRICHTUNG
DISPOSITIF DE COLLECTE DE GAZ

(30) Priority: 22.07.2022 KR 20220090849
(43) Date of publication of application: 10.07.2024
(73) Proprietor: LG Energy Solution, Ltd., Seoul 07335 (KR)
(72) Inventor: KANG, Hye Ok, Daejeon 34122 (KR); YUN, Heesun, Daejeon 34122 (KR); CHOI, Nak Hee, Daejeon 34122 (KR); LEE, Jong Keun, Daejeon 34122 (KR); PARK, Kwangyeon, Daejeon 34122 (KR)
(74) Representative: Goddar, Heinz J.
(86) International application number: PCT/KR2023/010359
(87) International publication number: WO 2024/019504

(56) References cited:
- WO-A1-2017/168864
- CN-A- 109 935 919
- JP-A- 2011 192 523
- KR-A- 20190 119 500
- KR-A- 20190 132 906
- KR-A- 20210 125 283
- KR-B1- 101 590 395
- US-A1- 2021 226 265

## Description

### Technical Field

The present application claims the benefit of priority based on Korean Patent Application No. 10-2022-0090849 filed on July 22, 2022.

The present disclosure relates to a gas collecting apparatus, and may be related to a gas collecting apparatus capable of collecting gas at an appropriate level of concentration for a battery having a case made of a rigid material of various specifications, in particular, a cylindrical battery.

### Background Art

In general, a secondary battery is a battery which can be used repeatedly through a discharging process in which chemical energy is converted into electrical energy and a charging process in the opposite direction to the discharging process and which includes nickelcadmium (Ni-Cd) batteries, nickel-metal hydride (Ni-MH) batteries, lithium-metal batteries, lithium-ion (Li-ion) batteries, and lithium-ion polymer batteries. Among these secondary batteries, lithium secondary batteries having high energy density and voltage, long cycle life, and a low self-discharge rate have been commercialized and widely used.

Depending on the reaction within the lithium secondary battery, various types of gases such as hydrogen, oxygen, nitrogen, carbon monoxide, carbon dioxide, hydrocarbons of CₙH₂ₙ₋₂ (n=2~5), CₙH₂ₙ (n=2~5), CₙH₂ₙ₊₂ (n=1~5), and other organic gas species, may be generated.

In addition, the lithium secondary battery degrades while generating a large amount of gas due to electrolyte decomposition as repeated charging and discharging progresses, and this aspect appears differently depending on the design and use form of the battery. Therefore, it is essential to infer a deterioration mechanism of a battery by analyzing gas generated inside the battery during a battery development process.

Therefore, it is very important to collect and accurately analyze the gas generated in the secondary battery. Various gases are generated during the operation of lithium ion batteries, and information on the composition and content of generated gases is useful for developing battery materials, optimizing battery manufacturing processes, and identifying causes of battery defects. To this end, it is important to develop a technology to collect the gas generated inside the secondary battery.

As a method for collecting gas generated inside a secondary battery, a method in which, when a battery is accommodated in a diffusion chamber with an airtight space formed therein, an opening for gas discharge is formed in the battery case, and then the gas discharged through the opening is diffused into the diffusion chamber, the diffused gas is collected in a separate gas collecting container is used. For high-concentration gas collection, it may be most desirable that there is no clearance between the battery and the inner space of the diffusion chamber.

Recently, batteries are used in a wide range of industrial fields, and depending on various battery application environments and conditions, performance required for batteries also varies, and batteries can be designed in various specifications.

Conventionally, in order to design various batteries, it was difficult to newly manufacture or install each time a battery model is changed by individually using a diffusion chamber for gas collection according to the size of the battery. In addition, when there is no clearance between the battery and the inner space of the diffusion chamber, it was difficult to remove the battery from the diffusion chamber.

In addition, the concentration of the gas delivered to an analyzer or stored in the gas collecting container varies greatly depending on the volume of the diffusion chamber, the size of the battery (material to be analyzed), and the amount of gas generated, which affects the analysis result. In order to solve this problem, a means for adjusting the concentration of the gas delivered to the analyzer or the gas stored in the gas collecting container to a concentration optimized for analysis is required.

KR 2019 0132906 A describes an apparatus for collecting generated gas of a secondary battery used for charging and discharging. The apparatus comprises a jig which is a cylindrical jig for mounting a cylindrical secondary battery, including a jig body part having outer and inner circumferential surfaces, an upper jig part positioned on an upper portion of the jig body part, and forming a space for mounting the cylindrical secondary battery together with the jig body part, and a lower jig part positioned at a lower portion of the jig body part, and forming a space for mounting the cylindrical secondary battery with the jig body part; a punching part provided at an upper portion of the upper jig part, and forming a groove in the cylindrical secondary battery; a connection part for connecting gas diffused from the cylindrical secondary battery to an acceptable manifold part, a collecting tube, or a gas analyzing device at a side surface of the jig body part; and charging and discharging terminals electrically connected to the cylindrical secondary battery. The cylindrical secondary battery may have a discharge capacity in the range of 1,000 to 5,000 mAh.

### Disclosure of the Invention

The present invention is defined according to the subject matter of the appended independent claim. Particular embodiments are given by the additional features of the appended dependent claims.

### Technical Goals

The present disclosure relates to a gas collecting apparatus, and is to provide a gas collecting apparatus capable of collecting gas at an appropriate level of concentration for a battery having a case made of a rigid material of various specifications, in particular, a cylindrical battery.

Technical objects to be achieved by the present disclosure are not limited to the technical objects mentioned above, and other technical objects not mentioned will be clearly understood by those skilled in the art from the description below.

### Advantageous Effects

A gas collecting apparatus of the present disclosure may have improved gas collection accuracy and convenience for an analyst by automating gas collection and high-concentration gas dilution.

The gas collecting apparatus of the present disclosure may be one in which batteries are easily installed, removed, or replaced when collecting gas.

The gas collecting apparatus of the present disclosure may detect whether punching is completed by depth of a punching needle or an amount of pressure change to prevent a battery internal short circuit, thereby improving stability during gas collection.

The gas collecting apparatus of the present disclosure may be one in which the concentration of the gas to be collected may be easily adjusted according to a gas analysis target, situation, and conditions during gas collection. Specifically, the gas collecting apparatus of the present disclosure may selectively collect gas from high concentration to low concentration, and may measure the degree of dilution or concentration.

### Brief Description of Drawings

FIG. 1 is a perspective view illustrating a gas collecting apparatus according of the present disclosure.
FIG. 2 is a cross-sectional view illustrating a cross section A-A of FIG. 1.
FIG. 3 is a cross-sectional view illustrating a cross section B-B of FIG. 1.
FIG. 4 is a cross-sectional view illustrating an operation of a punching unit.
FIG. 5 is a cross-sectional view illustrating separation of an inner jig.
FIG. 6 is a perspective view illustrating an inner jig.
FIG. 7 is a block diagram illustrating a gas collecting apparatus of the present disclosure.
FIG. 8 is a block diagram illustrating a gas collecting method of the present disclosure.

### Best Mode for Carrying Out the Invention

A gas collecting apparatus of the present disclosure includes
an inner jig with a battery seating groove into which a battery is inserted formed on an upper surface;
an outer housing accommodating the inner jig therein so that an outer circumferential surface of the inner jig adheres to its inner circumferential surface;
a gas discharge pipe connected to the outer housing and configured to deliver gas generated from the battery to a gas collecting pipe or a gas analyzer provided outside the outer housing;
a punching unit located at an upper end of the outer housing and forming a perforated hole for gas discharge on an upper surface of the battery; and
a punching driving unit configured to provide a driving force for driving the punching unit in a vertical direction.

**In** the gas collecting apparatus of the present disclosure, the punching unit includes a punching needle which is inserted inside the outer housing through a needle insertion hole provided at the upper end of the outer housing, a lower end of the punching needle perforating the upper surface of the battery to form the perforated hole on the upper surface of the battery, a punching rod configured to move in the vertical direction by the punching driving unit, an upper end of the punching needle being coupled to a lower end of the punching rod, and a sealing cover configured to surround an outer circumferential surface of the punching rod, a lower end of the sealing cover being coupled to the upper end of the outer housing.

In the gas collecting apparatus of the present disclosure, the sealing cover may be provided as bellows extending or contracting in the vertical direction, an upper end of the sealing cover may be coupled to a moving member of the punching driving unit together with an upper end of the punching rod, and the lower end of the sealing cover may cover the needle insertion hole and be coupled to the upper end of the outer housing.

**In** the gas collecting apparatus of the present disclosure, the punching driving unit includes a moving member to which an upper end of the sealing cover and an upper end of the punching rod are fixed, a support member fixed to an outer circumferential surface of the outer housing, and a driving means fixed to the support member and configured to move the moving member in the vertical direction.

In the gas collecting apparatus of the present disclosure, the driving means may include a shaft extending in the vertical direction and provided with a screw thread on its outer circumferential surface, and a motor configured to rotate the shaft while being supported by the support member, wherein in the moving member, a shaft insertion hole into which the shaft is inserted is formed, and on an inner circumferential surface of the shaft insertion hole, a screw thread engaging with the screw thread of the shaft is formed.

In the gas collecting apparatus of the present disclosure, the outer housing may include an upper housing in which an upper end of the inner jig is accommodated, and a lower housing in which a lower end of the inner jig is accommodated, wherein on an upper surface of the upper housing, the needle insertion hole is formed, and the punching unit is coupled to the upper surface of the upper housing while covering the needle insertion hole from an outer side of the upper housing.

In the gas collecting apparatus of the present disclosure, the inner jig may include an upper jig provided with a battery insertion hole into which an upper end of the battery is inserted, and a lower jig provided with a battery insertion groove into which a lower end of the battery is inserted on an upper surface, wherein the battery insertion hole is formed to penetrate the upper jig in the vertical direction, an upper opening of the battery insertion hole faces the needle insertion hole in an inner side of the upper housing, and a lower opening of the battery insertion hole faces an inlet of the battery insertion groove.

In the gas collecting apparatus of the present disclosure, the gas discharge pipe may be coupled to a side surface of the lower housing, in the upper jig, a first flow path configured to deliver gas discharged from an inside of the battery through the perforated hole and diffused inside the upper housing to the inside of the lower housing may be formed, and in the lower jig, a second flow path configured to deliver the gas delivered through the first flow path to the gas discharge pipe may be formed.

In the gas collecting apparatus of the present disclosure, an upper surface of the upper jig may be spaced apart from a ceiling surface at a predetermined interval inside the upper housing, the upper jig and the lower jig may be provided in a column shape extending in the vertical direction, the first flow path may be formed as a groove extending from an upper end to a lower end of the upper jig on an outer surface of the upper jig, and the second flow path may be formed as a groove extending from an upper end of the lower jig to a height at which the gas discharge pipe is connected to the lower housing on an outer surface of the lower jig.

In the gas collecting apparatus of the present disclosure, the upper jig and the lower jig may be provided in a cylindrical shape extending in the vertical direction, and at a lower end of a side surface of the upper jig or the upper end of the lower jig, a first connection flow path provided as a ring-shaped groove along an outer circumferential surface may be formed, wherein a lower end of the first flow path and an upper end of the second flow path may be connected to the first connection flow path.

In the gas collecting apparatus of the present disclosure, a second connection flow path provided as a ring-shaped groove along the outer circumferential surface may be formed in the lower jig, a lower end of the second flow path may be connected to the second connection flow path, and a height of the second connection flow path may be the same as the height at which the gas discharge pipe is connected to the lower housing.

The gas collecting apparatus of the present disclosure may further include a pressure sensor configured to measure a pressure inside the outer housing, wherein the punching driving unit is operated based on a measured value of the pressure sensor.

The gas collecting apparatus of the present disclosure may further include
a manifold unit connected to the gas discharge pipe to receive the gas generated from the battery from the outer housing;
a dilution tank unit connected to the manifold unit to dilute the gas delivered to the manifold unit;
a vacuum pump unit connected to the manifold unit to form a vacuum in the dilution tank unit; and
a gas delivering pipe connected to the manifold unit to deliver the gas to the gas collecting pipe or the gas collecting apparatus,
wherein the pressure sensor is connected to the manifold unit.

The gas collecting apparatus of the present disclosure may include
a first valve provided in the gas discharge pipe to block or open a flow of gas flowing through the gas discharge pipe to the manifold unit;
a second valve configured to block or open a flow path between the dilution tank unit and the manifold unit;
a third valve configured to block or open a flow path between the vacuum pump unit and the manifold unit;
a fourth valve provided in the gas delivering pipe to block or open a flow of gas flowing through the gas delivering pipe to the gas collecting pipe or the gas collecting apparatus;
a gas exhaust pipe connected to the manifold unit to exhaust the gas delivered to the manifold unit to an outside; and
a fifth valve provided in the gas exhaust pipe to block or open a flow of gas exhausted to the outside through the gas exhaust pipe.
A gas collecting method using the gas collecting apparatus of the present disclosure may include
a vacuum forming step of forming a vacuum inside the outer housing and the dilution tank through the vacuum pump unit;
a perforated hole forming step of forming the perforated hole in the battery by the punching unit;
a pressure change value calculating step of calculating a pressure change value through the measured value of the pressure sensor;
a needle retracting step of retracting the needle when the pressure change value is greater than or equal to a set value; and
a gas collecting step of opening the fourth valve and delivering gas to the gas collecting pipe or the gas analyzer.

### Modes for Carrying Out the Invention

Hereinafter, embodiments according to the present disclosure will be described in detail with reference to the accompanying drawings. In this process, the size or shape of components shown in the drawings may be exaggerated for clarity and convenience of explanation. In addition, terms specifically defined in consideration of the configurations and operations of the present disclosure may vary according to the intentions or customs of users and operators. Definitions of these terms should be made based on the disclosures throughout this specification.

In the description of the present disclosure, it should be noted that the orientation or positional relationship indicated by the terms such as "center", "upper", "lower", "left", "right", "vertical", "horizontal", "inner side", "outer side", "one surface", or "other surface" is based on the orientation or positional relationship shown in the drawing or the orientation or positional relationship normally arranged when using the product of the present disclosure, and it is intended only for explanation and brief description of the present disclosure, and is not to be construed as limiting the present disclosure because it does not suggest or imply that the device or element shown must necessarily be configured or operated in a specific orientation with a specific orientation.

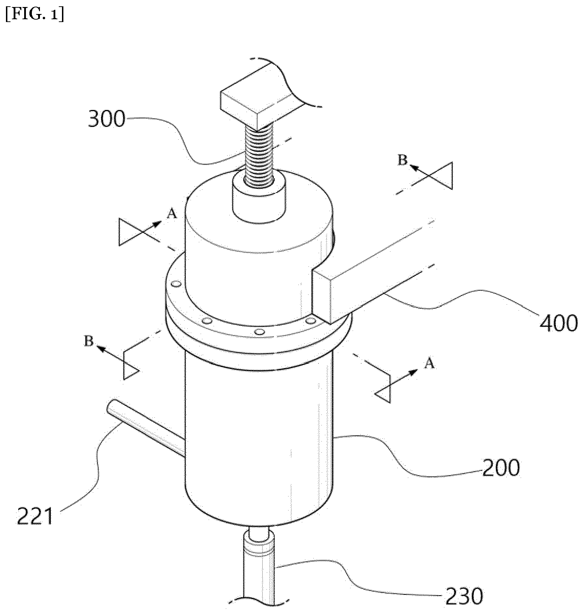

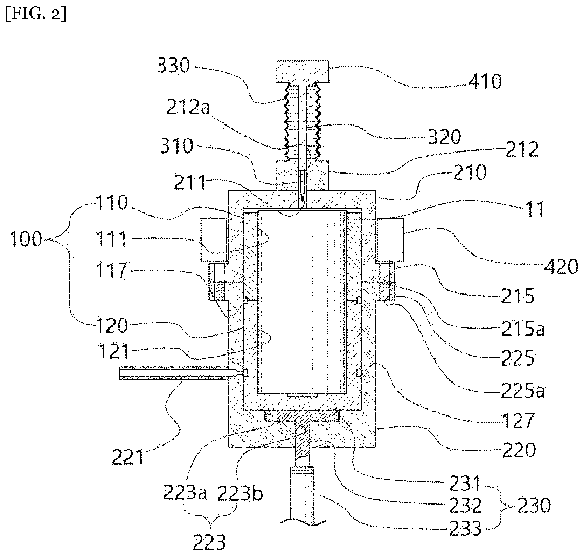

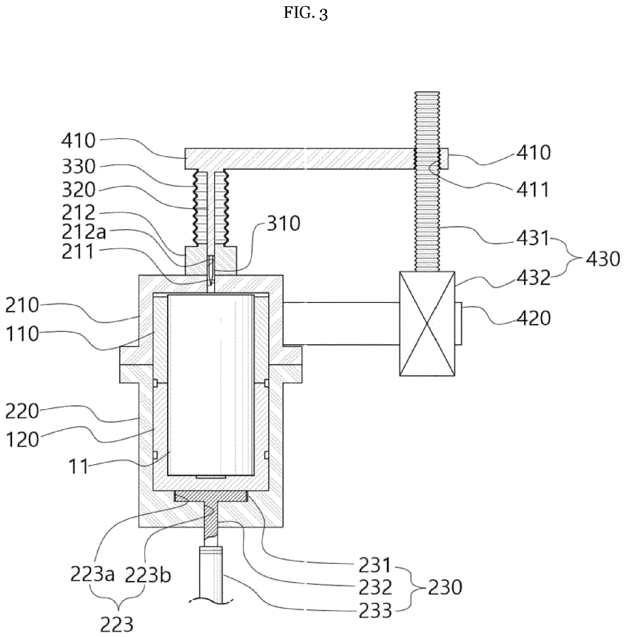

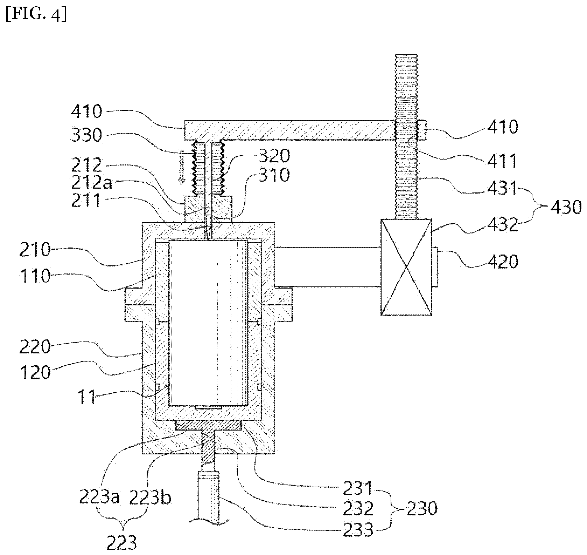

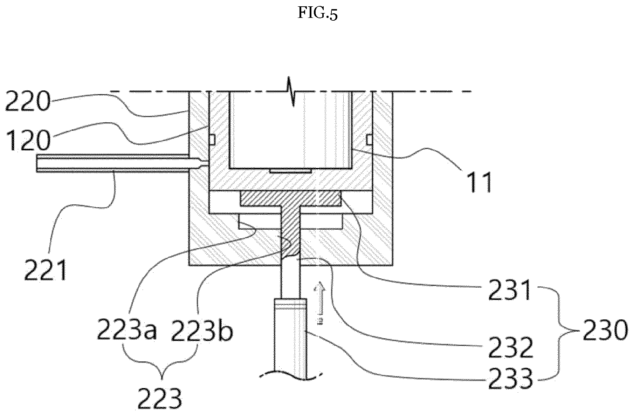

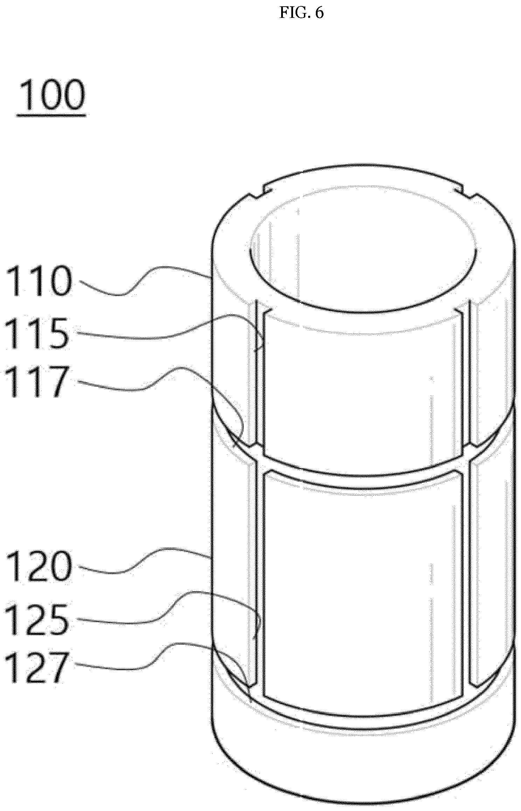

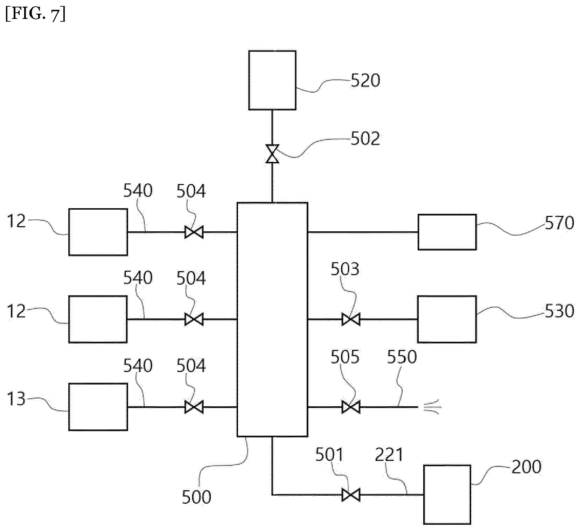

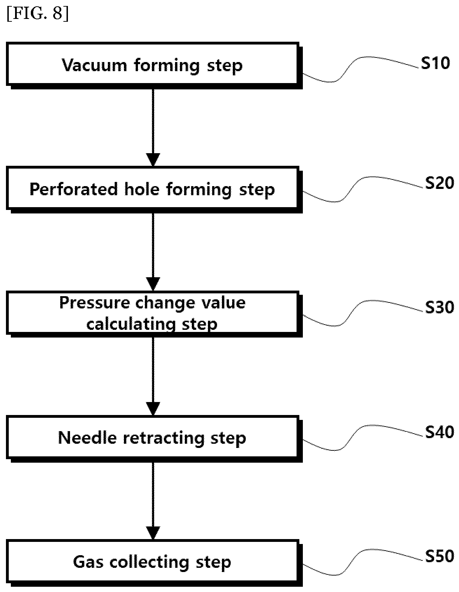

FIG. 1 is a perspective view illustrating a gas collecting apparatus according of the present disclosure. FIG. 2 is a cross-sectional view illustrating a cross section A-A of FIG. 1. FIG. 3 is a cross-sectional view illustrating a cross section B-B of FIG. 1. FIG. 4 is a cross-sectional view illustrating an operation of a punching unit 300. FIG. 5 is a cross-sectional view illustrating separation of an inner jig 100. FIG. 6 is a perspective view illustrating the inner jig 100. FIG. 7 is a block diagram illustrating a gas collecting apparatus of the present disclosure. FIG. 8 is a block diagram illustrating a gas collecting method of the present disclosure.

Hereinafter, with reference to FIGS. 1 to 8, the gas collecting apparatus of the present disclosure will be described in detail.

The gas collecting apparatus of the present disclosure may collect gas generated from an electrolyte solution, an electrode, or the like inside a battery 11. Specifically, when the battery 11 is charged and discharged or a specific condition (temperature, etc.) is given to the battery 11, various types of gases, such as hydrogen, oxygen, nitrogen, carbon monoxide, carbon dioxide, hydrocarbons of CₙH₂ₙ₋₂ (n=2~5), CₙH₂ₙ (n=2~5), CₙH₂ₙ₊₂ (n=1~5), and other organic gas species may be generated according to reactions within the battery 11. In order to quantitatively and qualitatively analyze such a gas, the gas collecting apparatus of the present disclosure may collect the gas.

In the gas collecting apparatus of the present disclosure, the battery 11 to be a gas collecting target may be a cylindrical battery. More specifically, it may be a battery in which gas is generated inside the battery case by charging/discharging or applying specific conditions (temperature, shock, vibration, etc.). In the gas collecting apparatus of the present disclosure, a perforated hole may be formed by perforating one end of the battery 11 in which gas is generated inside the battery case, and gas discharged through the perforated hole may be collected in the battery case.

As shown in FIGS. 1 to 3, the gas collecting apparatus of the present disclosure may include
the inner jig 100 with the battery 11 seating groove into which the battery 11 is inserted formed on an upper surface;
an outer housing 200 accommodating the inner jig 100 therein so that an outer circumferential surface of the inner jig 100 adheres to the inner circumferential surface of the outer housing;
a gas discharge pipe 221 connected to the outer housing 200 and configured to deliver gas generated from the battery 11 to a gas collecting pipe 12 or a gas analyzer 13 provided outside the outer housing 200;
the punching unit 300 located at an upper end of the outer housing 200 and forming a perforated hole for gas discharge on an upper surface of the battery 11; and
a punching driving unit 400 configured to provide a driving force for driving the punching unit 300 in a vertical direction.

The gas collecting apparatus of the present disclosure provides a plurality of inner jigs 100 having different inner diameters for each type of battery for battery 11 of various specifications, so that even if the specifications of the battery 11 are different, the volume of a space in which gas is diffused within the outer housing 200 may be the same.

In the gas collecting apparatus of the present disclosure, since the punching unit 300 configured to form the perforated hole in the battery 11 is automatically operated through the punching driving unit 400, precise and stable perforated hole formation may be possible. In the gas collecting apparatus of the present disclosure, the perforated hole may be formed by the punching unit 300 at one end of the cylindrical battery 11 and gas discharged from the battery 11 may be collected through the perforated hole.

As described above, in the gas collecting apparatus of the present disclosure, the battery to be a gas collecting target may be the cylindrical battery 11, and the inner jig 100 and the outer housing 200 may also be provided in a cylindrical shape.

As shown in FIGS. 1 to 3, the punching unit 300 may include a punching needle 310 which is inserted inside the outer housing 200 through a needle insertion hole 211 provided at the upper end of the outer housing 200, a lower end of the punching needle 310 perforating the upper surface of the battery 11 to form the perforated hole on the upper surface of the battery 11, a punching rod 320 configured to move in the vertical direction by the punching driving unit 400, an upper end of the punching needle 310 being coupled to a lower end of the punching rod 320, and a sealing cover 330 configured to surround an outer circumferential surface of the punching rod 320, a lower end of the sealing cover 330 being coupled to the upper end of the outer housing 200.

The punching needle 310 may be a rigid member having a sharp lower end. The punching needle 310 may be linearly movable in the upward and downward directions. When the punching needle 310 moves downward, the sharp lower end of the punching needle 310 may be inserted into the upper end of the battery 11 to form a perforated hole in the battery case.

The punching rod 320 may have a lower end coupled to the upper end of the punching needle 310 and move in the vertical direction together with the punching needle 310. The punching rod 320 may be provided in a bar shape extending in the vertical direction. The punching rod 320 may be made of a highly rigid chemical resistant material.

The sealing cover 330 may be provided as bellows extending or contracting in the vertical direction, an upper end of the sealing cover 330 may be coupled to a moving member 410 of the punching driving unit 400 together with an upper end of the punching rod 320, and the lower end of the sealing cover 330 may cover the needle insertion hole 211 and be coupled to the upper end of the outer housing 200. For example, the punching needle 310 may be provided in a cylindrical shape with a pointed lower end, and the punching rod 320 may be provided in a cylindrical shape having a larger diameter than the punching needle 310.

On the upper surface of the outer housing 200, a needle insertion hole 211 penetrating the ceiling surface of the outer housing 200 is formed, and the punching needle 310 may be inserted into the needle insertion hole 211. The punching needle 310 may form a perforated hole by approaching the upper end of the battery 11 located inside the outer housing 200 through the needle insertion hole 211.

On the upper surface of the outer housing 200, a guide member 212 configured to guide vertical movement of the punching rod 320 may be provided. In the guide member 212, a rod guide hole 212a is provided in the vertical direction of the guide member 212, and a punching rod 320 may be inserted into the rod guide hole 212a. The lower opening of the rod guide hole 212a may face the upper opening of the needle insertion hole 211. The outer diameter of the punching rod 320 and the inner diameter of the rod guide hole 212a may match.

The lower end of the sealing cover 330 may be coupled to the upper end of the guide member 212, and more specifically, the lower end of the sealing cover 330 may be coupled to the upper end of the guide member 212 while covering the upper opening of the rod guide hole 212a. The sealing cover 330 may prevent gas inside and outside the outer housing 200 from being ventilated through the rod guide hole 212a.

As shown in FIGS. 3 and 4, the punching driving unit 400 may include a moving member 410 to which an upper end of the sealing cover 330 and an upper end of the punching rod 320 are fixed, a support member 420 fixed to an outer circumferential surface of the outer housing 200, and a driving means 430 fixed to the support member 420 and configured to move the moving member 410 in the vertical direction.

The moving member 410 moves in the vertical direction through the driving force of the driving means, and more specifically, may transmit the driving force of the driving means 430 to the punching needle 310. The punching needle 310 may be fixed to the moving member 410 through the punching rod 320, and the punching needle 310 may move vertically along with the moving member 410 by the moving member 410 moving in the vertical direction. The moving member 410 may be provided in a bar shape extending in a direction perpendicular to the vertical direction, wherein the punching rod 320 and the upper end of the sealing cover may be coupled to one end of the moving member 410, and the driving means 430 may be coupled to the other end. The moving member 410 may be provided with a guide means (not shown) supported by the outer housing 200 and guiding the vertical movement of the moving member 410. The moving member 410 may move while sliding in the vertical direction while in contact with the guide means.

The support member 420 may be fixed to the outer housing 200. The support member 420 supports the driving means 430 and may prevent the driving means 430 from changing its relative location with respect to the outer housing 200.

As the separation distance of the moving member 410 relative to the support member 420 in the vertical direction is changed by the driving means 430, the punching needle may approach or be separated from the battery 11.

The driving means 430 may include a shaft 431 extending in the vertical direction and provided with a screw thread on its outer circumferential surface, and a motor 432 configured to rotate the shaft 431 while being supported by the support member, wherein in the moving member 410, a shaft insertion hole 411 into which the shaft 431 is inserted is formed, and on an inner circumferential surface of the shaft insertion hole 411, a screw thread engaging with the screw thread of the shaft 431 is formed.

The shaft 431 may extend in the vertical direction and rotate in the vertical direction, which is a longitudinal direction, as a rotation axis direction. The relative position of the shaft 431 and the support member 420 may be constant. Since the screw thread of the shaft insertion hole 411 provided in the moving member 410 is engaged with the screw thread of the shaft 431, when the shaft 431 rotates, the moving member 410 may move in the vertical direction.. When the shaft 431 rotates, the moving member 410 may be prevented from rotating together with the shaft 431 by the guide means.

The motor 432 may provide a driving force for rotating the shaft 431. Since the gas collecting apparatus of the present disclosure converts rotational motion into linear motion when the punching needle 310 moves, precise manipulation may be possible. A decrease in the controllability of the insertion depth of the punching needle 310 may cause an accident by causing a short circuit of the electrode. The gas collecting apparatus of the present disclosure may be capable of stable operation by preventing accidents by precisely manipulating the punching needle 310. The gas collecting apparatus of the present disclosure may measure the vertical height of the punching needle 310 by sensing the amount of rotation of the shaft 431. For example, an encoder, a control unit, etc., may be connected to the motor 432 to measure and record the amount of rotation of the shaft 431.

As shown in FIGS. 2 and 3, the outer housing 200 may include an upper housing 210 in which an upper end of the inner jig 100 is accommodated, and a lower housing 220 in which a lower end of the inner jig 100 is accommodated, wherein on an upper surface of the upper housing 210, the needle insertion hole 211 is formed, and the punching unit 300 is coupled to the upper surface of the upper housing 210 while covering the needle insertion hole 211 from an outer side of the upper housing 210.

A space in which the lower end is opened is provided inside the upper housing 210, and a space in which the upper end of the lower housing 220 is opened is provided, and by the upper housing 210 and the lower housing 220 being coupled so that the lower end of the upper housing 210 and the upper end of the lower housing 220 contact each other, the two spaces may be formed as one closed space, and the inner jig 100 may be accommodated in the closed space.

The upper housing 210 and the lower housing 220 may be provided in a cylindrical shape extending in the vertical direction. At this time, a ring-shaped first protrusion 215 protruding in the radial direction may be formed at the lower end of the outer circumferential surface of the upper housing 210, and a ring-shaped second protrusion 225 protruding in the radial direction may be formed at the upper end of the outer circumferential surface of the lower housing 220.

The lower side surface of the first protrusion 215 and the upper side surface of the second protrusion 225 may come into close contact with each other to improve airtightness of a closed space formed by coupling the upper housing 210 and the lower housing 220. A sealing member such as an O-ring or a gasket may be inserted between the first protrusion 215 and the second protrusion 225 to further improve airtightness.

In order to fix the coupling between the upper housing 210 and the lower housing 220, the first protrusion 215 is provided with a plurality of first fixing holes 215a, and the second protrusion 225 is provided with a plurality of second fixing holes 225a, and a fixing member (not shown) may be simultaneously inserted into the first fixing hole 215a and the second fixing hole 225a to fix the coupling between the upper housing 210 and the lower housing 220. The fixing member may be a bolt provided with a screw thread, and a screw thread for screwing with the fixing member may be provided on an inner circumferential surface of the first fixing hole 215a or the second fixing hole 225a.

As another embodiment for fixing the coupling between the upper housing 210 and the lower housing 220, by providing a coupling means (not shown) for fixing the position of one of the upper housing 210 and the lower housing 220 and pressing the other unfixed one toward the fixed one, the coupling between the upper housing 210 and the lower housing 220 may be fixed. For example, the lower housing 220 may be fixed to a support (not shown), and a pneumatic actuator may be connected to the upper end of the upper housing 210 as the coupling means. The pneumatic actuator applies downward force to the upper housing 210, and the lower end of the upper housing 210 may be fixed in close contact with the upper end of the lower housing 220 by the force applied by the actuator.

The upper housing 210 and the lower housing 220 may be made of stainless-steel material.

The inner jig 100 may include an upper jig 110 provided with a battery insertion hole 111 into which an upper end of the battery 11 is inserted, and a lower jig 120 provided with a battery insertion groove 121 into which a lower end of the battery 11 is inserted on an upper surface, wherein the battery insertion hole 111 is formed to penetrate the upper jig 110 in the vertical direction, an upper opening of the battery insertion hole 111 faces the needle insertion hole 211 in an inner side of the upper housing 210, and a lower opening of the battery insertion hole 111 faces an inlet of the battery insertion groove 121.

The upper jig 110 and the lower jig 120 may be provided in a cylindrical shape extending in the vertical direction. The outer diameters of the upper jig 110 and the lower jig 120 coincide with the inner diameters of the upper housing 210 and the lower housing 220, so that the outer circumferential surface of the upper jig 110 and the inner circumferential surface of the upper housing 210 may come into close contact with each other, and the outer circumferential surface of the lower jig 120 and the inner circumferential surface of the lower housing 220 may come into close contact with each other.

As shown in FIGS. 2 to 4, the vertical length of the upper jig 110 may be longer than the vertical length of the inner space of the upper housing 210, and the vertical length of the lower jig 120 may be shorter than the vertical length of the inner space of the lower housing 220. Accordingly, the coupling portion between the upper jig 110 and the lower jig 120 may be located lower than the coupling portion between the upper housing 210 and the lower housing 220. Therefore, in the process of coupling the inner jig 100 to the outer housing 200, after the upper jig 110 and the lower jig 120 are simultaneously fixed to the lower housing 220, the upper housing 210 may be stably coupled to the upper end of the lower housing 220.

Inside the upper jig 110, a battery insertion hole 111 may be provided as a space for accommodating the battery 11. Accordingly, the inner space of the upper jig 110 may be a space in which both the upper and lower ends are open. The upper opening of the battery insertion hole 111 may allow the punching needle 310 to access the upper end of the battery 11, and the lower opening of the battery insertion hole 111 may be for merging the space formed by the battery insertion hole 111 and the space formed by the battery insertion groove 121 into one space.

Inside the lower jig 120, a battery insertion groove 121 may be provided as a space for the battery 11 to be accommodated. The opening of the battery insertion groove 121 may be provided on the upper surface of the lower jig 120. Therefore, when the upper jig 110 and the lower jig 120 are coupled so that the lower end of the upper jig 110 and the upper end of the lower jig 120 face each other, the space of the battery insertion hole 111 and the space of the battery insertion groove 121 may be combined to form a single space for accommodating one battery 11.

The upper jig 110 and the lower jig 120 may be made of Teflon material.

A jig separator 230 for separating the lower jig 120 from the lower housing 220 may be provided at the lower end of the lower housing 220.

As shown in FIG. 5, the jig separator 230 may include a disk-shaped support plate 231 whose upper surface contacts the lower surface of the lower jig 120, a support bar 232 whose upper end is coupled to the lower surface of the support plate 231 and whose lower end protrudes to the outside of the lower housing 220 through a penetration hole 223 provided on the bottom surface of the lower housing 220, and a pneumatic cylinder 233 coupled to the lower end of the support bar 232 to move the support plate 231 and the support bar 232 in the vertical direction.

The support plate 231 may be provided in a disk shape having a plane perpendicular to the vertical direction, and the support bar 232 may be provided in a bar shape extending in the vertical direction.

The diameter of the support plate 231 may be formed larger than the outer diameter of the support bar 232, so that the lower jig 120 may be raised upward by supporting the lower surface of the lower jig 120 with a larger area than the support bar 232 directly contacting the lower jig 120.

The penetration hole 223 may include a first penetration hole 223a having an upper opening located inside the lower housing 220, and a second penetration hole 223b having a lower opening located outside the lower housing 220, wherein the lower opening of the first penetration hole 223a is connected to the upper opening of the second penetration hole 223b. The inner diameter of the first penetration hole 223a may be greater than or equal to the diameter of the support plate 231 so that the support plate 231 may be completely buried in the bottom surface of the lower housing 220. The length of the first penetration hole 223a in the vertical direction may be greater than or equal to the thickness of the support plate 231. The inner diameter of the second penetration hole 223b may be formed to be the same as the outer diameter of the support bar 232, so that the outer circumferential surface of the support bar 232 is completely brought into close contact with the inner circumferential surface of the second penetration hole 223b to improve airtightness of the inner space of the outer housing 200.

As shown in FIG. 6, the gas discharge pipe 221 may be coupled to a side surface of the lower housing 220, in the upper jig 110, a first flow path 115 configured to deliver gas discharged from an inside of the battery 11 through the perforated hole and diffused inside the upper housing 210 to the inside of the lower housing 220 may be formed, and in the lower jig 120, a second flow path 125 configured to deliver the gas delivered through the first flow path 115 to the gas discharge pipe 221 may be formed.

An upper surface of the upper jig 110 may be spaced apart from a ceiling surface at a predetermined interval inside the upper housing 210, the upper jig 110 and the lower jig 120 may be provided in a column shape extending in the vertical direction, the first flow path 115 may be formed as a groove extending from an upper end to a lower end of the upper jig 110 on an outer surface of the upper jig 110, and the second flow path 125 may be formed as a groove extending from an upper end of the lower jig 120 to a height at which the gas discharge pipe 221 is connected to the lower housing 220 on an outer surface of the lower jig 120.

The upper jig 110 and the lower jig 120 may be provided in a cylindrical shape extending in the vertical direction, and at a lower end of a side surface of the upper jig 110 or the upper end of the lower jig 120, a first connection flow path 117 provided as a ring-shaped groove along an outer circumferential surface may be formed, wherein a lower end of the first flow path 115 and an upper end of the second flow path 125 is connected to the first connection flow path 117. By providing the first connection flow path 117, even if the upper jig 110 and the lower jig 120 are not assembled so that the position of the lower end of the first flow path 115 completely matches the position of the upper end of the second flow path 125, gas in the first flow path 115 may flow to the second flow path 125 through the first connection flow path 117.

A second connection flow path 127 provided as a ring-shaped groove along the outer circumferential surface may be formed in the lower jig 120, a lower end of the second flow path 125 may be connected to the second connection flow path 127, and a height of the second connection flow path 127 may be the same as the height at which the gas discharge pipe 221 is connected to the lower housing 220. By providing the second connection flow path 127, even if the position where the gas discharge pipe 221 is connected to the lower housing 220 does not face the second flow path 125, gas in the second flow path 125 may be delivered to the gas discharge pipe 221 through the second flow path 127.

The gas discharge pipe 221 is provided as a tube, hose, or pipe, and may be used to deliver gas generated in the battery 11 to the gas collecting pipe 12 or the gas analyzer 13. The gas collecting pipe 12 may be a container in which an airtight sealed space is formed to store gas. The gas analyzer 13 may be a device that receives gas and analyzes gas components and amounts. In other words, in order to qualitatively or quantitatively analyze the gas generated from the battery 11, the gas collecting apparatus of the present disclosure may be for directly delivering the gas to the gas analyzer 13 or storing in the gas collecting pipe 12.

The gas collecting apparatus of the present disclosure may further include
a pressure sensor 570 configured to measure a pressure inside the outer housing 200, wherein the punching driving unit 400 is operated based on a measured value of the pressure sensor 570.

In the gas collecting apparatus of the present disclosure, the battery 11 to be a gas collecting target may be in a state in which gas is generated therein. Therefore, when a perforated hole is formed in the battery 11 by the punching needle 310, gas inside the battery 11 diffuses into the outer housing 200 through the perforated hole, and the pressure in the inner space of the outer housing 200 may be increased. The gas collecting apparatus of the present disclosure may recognize the formation of the perforated hole by detecting this, and lift the punching needle 310 to separate the punching needle 310 from the battery 11.

As shown in FIG. 7, the gas collecting apparatus of the present disclosure may further include
a manifold unit 500 connected to the gas discharge pipe 221 to receive the gas generated from the battery 11 from the outer housing 200;
a dilution tank unit 520 connected to the manifold unit 500 to dilute the gas delivered to the manifold unit 500;
a vacuum pump unit 530 connected to the manifold unit 500 to form a vacuum in the dilution tank unit 520; and
a gas delivering pipe 540 connected to the manifold unit 500 to deliver the gas to the gas collecting pipe 12 or the gas collecting apparatus, wherein the pressure sensor 570 is connected to the manifold unit 500.

The manifold unit 500 may connect the flow paths to which they are connected.

The dilution tank unit 520 may be a container in which an airtight space is formed inside. Specifically, the gas collecting apparatus of the present disclosure may make the internal pressure of the gas collecting pipe 12 or the gas collecting apparatus lower than the inside of the outer housing 200 through the vacuum pump unit 530 to move the gas through gas diffusion through a pressure difference. At this time, by connecting the dilution tank unit 520 to the flow path connecting the outer housing 200 and the gas collecting pipe 12 or the gas analyzer 13 through the manifold unit 500, and expanding the space in which the gas diffuses when the gas moves through the dilution tank unit 520, high concentration gases can be diluted to a level suitable for gas analysis.

The gas collecting apparatus of the present disclosure may basically be capable of collecting high-concentration gas by filling the inner space of the outer housing 200 through the inner jig 100, and may be adjustable to a gas concentration suitable for analysis through the dilution tank unit 520. In other words, the gas collecting apparatus of the present disclosure has a high degree of freedom in adjusting the gas concentration.

Before gas is discharged from the outer housing 200, a vacuum may be previously formed inside the dilution tank unit 520 through a vacuum pump.

The vacuum pump unit 530 may be used to form a negative pressure in the gas collecting pipe 12, the gas analyzer 13, or the dilution tank.

The gas delivering pipe 540 is a pipe for forming a flow path, and may be configured to deliver gas between the manifold unit and the gas collecting pipe 12 or the gas analyzer 13.

As shown in FIG. 7, the gas collecting apparatus of the present disclosure may include
a first valve 501 provided in the gas discharge pipe 221 to block or open a flow of gas flowing through the gas discharge pipe 221 to the manifold unit 500;
a second valve 502 configured to block or open a flow path between the dilution tank unit 520 and the manifold unit 500;
a third valve 503 configured to block or open a flow path between the vacuum pump unit 530 and the manifold unit 500;
a fourth valve 504 provided in the gas delivering pipe 540 to block or open a flow of gas flowing through the gas delivering pipe 540 to the gas collecting pipe 12 or the gas collecting apparatus;
a gas exhaust pipe 550 connected to the manifold unit 500 to exhaust the gas delivered to the manifold unit 500 to the outside; and
a fifth valve 505 provided in the gas exhaust pipe 550 to block or open a flow of gas exhausted to the outside through the gas exhaust pipe 550.

The first valve 501, the second valve 502, the third valve 503, the fourth valve 504, and the fifth valve 505 may be electronic valves. The electronic valve is connected to the control unit and may be controlled through the control unit. The control unit may be a processor. A pressure sensor 570, a motor 432, an encoder, etc., are connected to the control unit, and based on the pressure value or the operating value of the punching unit 300, etc., the first valve 501, the second valve 502, the third valve 503, the fourth valve 504 and the fifth valve 505 may be operated.

The gas exhaust pipe 550 has one end connected to the manifold unit 500 and the other end open to the atmosphere or connected to a scrubber, and may be used in a temporary high pressure relief or purge mode. In this case, the fifth valve 505 may be a relief valve.

As shown in FIG. 8, a gas collecting method of the present disclosure may include
a vacuum forming step of forming a vacuum inside the outer housing and the dilution tank through the vacuum pump unit;
a perforated hole forming step of forming the perforated hole in the battery 11 by the punching unit 300;
a pressure change value calculating step of calculating a pressure change value through the measured value of the pressure sensor 570;
a needle retracting step of retracting the needle when the pressure change value is greater than or equal to a set value; and
a gas collecting step of opening the fourth valve 504 and delivering gas to the gas collecting pipe 12 or the gas analyzer 13.

The gas collecting method of the present disclosure may further include a battery 11 mounting step before the vacuum forming step.

In the battery 11 mounting step, the battery 11 may have an upper end inserted into the upper jig 110 and a lower end inserted into the lower jig 120 to be coupled with the inner jig 100. At this time, the battery 11 may be coupled to the inner jig 100 so that the lower end of the upper jig 110 and the upper end of the lower jig 120 come into close contact.

In the battery 11 mounting step, the upper housing 210 and the lower housing 220 may be respectively covered on the upper end and lower end of the inner jig 100 coupled with the battery 11, respectively, and the upper housing 210 and the lower housing 220 may be coupled to each other so that the lower end of the upper housing 210 and the upper end of the upper housing 210 come into close contact with each other, so that the inner jig 100 may be accommodated inside the outer housing 200.

In the vacuum forming step, the vacuum may be formed by operating the vacuum pump unit 530 while the first valve 501, the second valve 502, and the third valve 503 are open. The fourth valve 504 is also opened in the vacuum forming step, so that a vacuum may be formed in the gas collecting space provided in the gas collecting pipe 12 or the gas analyzer 13 as well. The fifth valve 505 may be closed.

In the perforated hole forming step, the punching driving unit 400 may lower the punching needle 310 to form a perforated hole in the battery 11. Here, the second valve 502 and the third valve 503 may be closed.

In the pressure change value calculating step, the control unit may receive a pressure measured value from the pressure sensor 570 and calculate the pressure change value over time.

In the gas collecting step, the fourth valve 504 is opened, and the collected gas may be diluted by opening the second valve 502 if necessary.

More specifically, in the gas collecting apparatus of the present disclosure, the dilution tank unit 520 and the second valve 502 are provided in plurality in pairs, and in each of the plurality of dilution tank units 520, the flow of gas with the manifold unit 500 is independently opened or closed by the plurality of second valves 502, and the gas collecting step may include
a first collecting step of opening the fourth valve 504 in a state in which all of the plurality of second valves 502 are closed,
a first dilution step of, if the measured value of the pressure sensor 570 is less than or equal to the set pressure value after a first set time, completing the collection by determining as a normal concentration, and if greater than or equal to the set pressure value, opening some or all of the plurality of second valves 502 by determining as a high concentration,
a second dilution step of, if the measured value of the pressure sensor 570 is less than or equal to the set pressure value after a second set time, completing the collection by determining as a normal concentration, and if greater than or equal to the set pressure value, forming a vacuum by opening the third valve 503 in a state in which the fourth valve 504 is closed together with some of the plurality of second valves 502 opened in the first dilution step by determining as a high concentration, and
a second collecting step of closing the third valve 503 and opening the fourth valve and some or all of the plurality of second valves 502 closed in the second dilution step.

The first set time and the second set time may be determined in consideration of a gas diffusion rate, and the set pressure may be determined by specifications of the gas analyzer that analyzes the collected gas.

In the first dilution step, the second dilution step, and the second collecting step, control of selective opening or closing of the plurality of second valves 502 may be determined in consideration of the capacity of the dilution tank 520, the measured value of the pressure sensor, and the like.

After the gas collecting step, a purge step and a separation step may be further included.

In the purge step, the inside of the outer housing 200 may be purged by injecting a purge gas into the manifold unit 500 in a state where the first valve 501 and the fifth valve 505 are open.

In the separation step, after the upper housing 210 and the upper jig 110 are separated from the battery 11, the lower jig 120 may be discharged from the lower housing 220 by operating the jig separator 230.

Although embodiments according to the present disclosure have been described above, they are only illustrative and those skilled in the art will understand that various modifications and embodiments of equivalent range are possible therefrom. Therefore, the true technical protection scope of the present disclosure should be defined by the following claims.

### <Explanation of Symbols>

11...Battery, 12...Gas collecting pipe 13...Gas analyzer, 100...Inner jig, 110...Upper jig, 111...Battery insertion hole, 115...First flow path, 117...First connection flow path, 120...Lower jig, 121...Battery insertion groove, 125...Second flow path, 127...Second connection flow path, 200...Outer housing, 210...Upper housing, 211...Needle insertion hole, 212...Guide member, 212a...Rod guide hole, 215...First protrusion, 215a...First fixing hole, 220...Lower housing, 221...Gas discharge pipe, 223...Penetration hole, 223a...First penetration hole, 223b...Second penetration hole, 225...Second protrusion, 225a...Second fixing hole, 230...Jig separator, 231...Support plate, 232...Support bar, 233...Pneumatic cylinder, 300...Punching unit, 310...Punching needle, 320...Punching rod, 330...Sealing cover, 400...Punching driving unit, 410... Moving member, 411...Shaft insertion hole, 420...Support member, 430...Driving means, 431...Shaft, 432...Motor, 500... Manifold unit, 501...First valve, 502...Second valve, 503... Third valve, 504...Fourth valve, 505...Fifth valve, 520...Dilution tank unit, 530...Vacuum pump unit, 540...Gas delivering pipe, 550...Gas exhaust pipe, 570...Pressure sensor

### Industrial Applicability

A gas collecting apparatus of the present disclosure may have improved gas collection accuracy and convenience for the analyst by automating gas collection and high-concentration gas dilution.

The gas collecting apparatus of the present disclosure may be one in which batteries are easily installed, removed, or replaced when collecting gas.

The gas collecting apparatus of the present disclosure may detect whether punching is completed by the depth of the punching needle or the amount of pressure change to prevent a battery internal short circuit, thereby improving stability during gas collection.

The gas collecting apparatus of the present disclosure may be one in which the concentration of the gas to be collected may be easily adjusted according to the gas analysis target, situation, and conditions during gas collection. Specifically, The gas collecting apparatus of the present disclosure may selectively collect gas from high concentration to low concentration, and may measure the degree of dilution or concentration.

## Claims

1. A gas collecting apparatus comprising:
an inner jig (100) with a battery seating groove into which a battery is inserted formed on an upper surface;
an outer housing (200) accommodating the inner jig (100) therein so that an outer circumferential surface of the inner jig (100) adheres to its inner circumferential surface;
a gas discharge pipe (221) connected to the outer housing (200) and configured to deliver gas generated from the battery to a gas collecting pipe (12) or a gas analyzer (13) provided outside the outer housing (200);
a punching unit (300) located at an upper end of the outer housing (200) and forming a perforated hole for gas discharge on an upper surface of the battery; and
a punching driving unit (400) configured to provide a driving force for driving the punching unit (300) in a vertical direction,
wherein the punching unit (300) comprises:
a punching needle (310) which is inserted inside the outer housing (200) through a needle insertion hole (211) provided at the upper end of the outer housing (200), a lower end of the punching needle (310) perforating the upper surface of the battery to form the perforated hole on the upper surface of the battery;
a punching rod (320) configured to move in the vertical direction by the punching driving unit (400), an upper end of the punching needle (310) being coupled to a lower end of the punching rod (320); and
a sealing cover (330) configured to surround an outer circumferential surface of the punching rod (320), a lower end of the sealing cover (330) being coupled to the upper end of the outer housing (200),
wherein the punching driving unit (400) comprises:
a moving member (410) to which an upper end of the sealing cover (330) and an upper end of the punching rod (320) are fixed;
a support member (420) fixed to an outer circumferential surface of the outer housing (200); and
a driving means (430) fixed to the support member (420) and configured to move the moving member (410) in the vertical direction.

2. The gas collecting apparatus of claim 1, wherein the sealing cover (330) is provided as bellows extending or contracting in the vertical direction,
an upper end of the sealing cover (330) is coupled to a moving member (410) of the punching driving unit (400) together with an upper end of the punching rod (320), and
the lower end of the sealing cover (330) covers the needle insertion hole (211) and is coupled to the upper end of the outer housing (200).

3. The gas collecting apparatus of claim 1, wherein the driving means (430) comprises:
a shaft (431) extending in the vertical direction and provided with a screw thread on its outer circumferential surface; and
a motor (432) configured to rotate the shaft (431) while being supported by the support member (420), and
wherein in the moving member (410), a shaft (431) insertion hole into which the shaft (431) is inserted is formed, and
on an inner circumferential surface of the shaft (431) insertion hole, a screw thread engaging with the screw thread of the shaft (431) is formed.

4. The gas collecting apparatus of claim 1, wherein the outer housing (200) comprises:
an upper housing (210) in which an upper end of the inner jig (100) is accommodated; and
a lower housing (220) in which a lower end of the inner jig (100) is accommodated, and
wherein on an upper surface of the upper housing (210), the needle insertion hole (211) is formed, and
the punching unit (300) is coupled to the upper surface of the upper housing (210) while covering the needle insertion hole (211) from an outer side of the upper housing (210).

5. The gas collecting apparatus of claim 4, wherein the inner jig (100) comprises:
an upper jig (110) provided with a battery insertion hole (111) into which an upper end of the battery is inserted; and
a lower jig (120) provided with a battery insertion groove (121) into which a lower end of the battery is inserted on an upper surface, and
wherein the battery insertion hole (111) is formed to penetrate the upper jig (110) in the vertical direction,
an upper opening of the battery insertion hole (111) faces the needle insertion hole (211) in an inner side of the upper housing (210), and
a lower opening of the battery insertion hole (111) faces an inlet of the battery insertion groove (121).

6. The gas collecting apparatus of claim 5, wherein the gas discharge pipe (221) is coupled to a side surface of the lower housing (220),
in the upper jig (110), a first flow path (115) configured to deliver gas discharged from an inside of the battery through the perforated hole and diffused inside the upper housing (210) to the inside of the lower housing (220) is formed, and
in the lower jig (120), a second flow path (125) configured to deliver the gas delivered through the first flow path (115) to the gas discharge pipe (221) is formed.

7. The gas collecting apparatus of claim 6, wherein an upper surface of the upper jig (110) is spaced apart from a ceiling surface at a predetermined interval inside the upper housing (210),
the upper jig (110) and the lower jig (120) are provided in a column shape extending in the vertical direction,
the first flow path (115) is formed as a groove extending from an upper end to a lower end of the upper jig (110) on an outer surface of the upper jig (110), and
the second flow path (125) is formed as a groove extending from an upper end of the lower jig (120) to a height at which the gas discharge pipe (221) is connected to the lower housing (220) on an outer surface of the lower jig (120).

8. The gas collecting apparatus of claim 7, wherein the upper jig (110) and the lower jig (120) are provided in a cylindrical shape extending in the vertical direction,
at a lower end of a side surface of the upper jig (110) or the upper end of the lower jig (120), a first connection flow path (117) provided as a ring-shaped groove along an outer circumferential surface is formed, and
a lower end of the first flow path (115) and an upper end of the second flow path (125) are connected to the first connection flow path (117).

9. The gas collecting apparatus of claim 8, wherein a second connection flow path (127) provided as a ring-shaped groove along the outer circumferential surface is formed in the lower jig (120),
a lower end of the second flow path (125) is connected to the second connection flow path (127), and
a height of the second connection flow path (127) is the same as the height at which the gas discharge pipe (221) is connected to the lower housing (220).

10. The gas collecting apparatus of claim 1, further comprising:
a pressure sensor (570) configured to measure a pressure inside the outer housing (200),
wherein the punching driving unit (400) is operated based on a measured value of the pressure sensor (570).

11. The gas collecting apparatus of claim 10, further comprising:
a manifold unit (500) connected to the gas discharge pipe (221) to receive the gas generated from the battery from the outer housing (200);
a dilution tank (520) unit connected to the manifold unit (500) to dilute the gas delivered to the manifold unit (500);
a vacuum pump unit (530) connected to the manifold unit (500) to form a vacuum in the dilution tank (520) unit; and
a gas delivering pipe (540) connected to the manifold unit (500) to deliver the gas to the gas collecting pipe (12) or the gas collecting apparatus,
wherein the pressure sensor (570) is connected to the manifold unit (500).

12. The gas collecting apparatus of claim 11, comprising:
a first valve (501) provided in the gas discharge pipe (221) to block or open a flow of gas flowing through the gas discharge pipe (221) to the manifold unit (500);
a second valve (502) configured to block or open a flow path between the dilution tank (520) unit and the manifold unit (500);
a third valve (503) configured to block or open a flow path between the vacuum pump unit (530) and the manifold unit (500);
a fourth valve (504) provided in the gas delivering pipe (540) to block or open a flow of gas flowing through the gas delivering pipe (540) to the gas collecting pipe (12) or the gas collecting apparatus;
a gas exhaust pipe (550) connected to the manifold unit (500) to exhaust the gas delivered to the manifold unit (500) to an outside; and
a fifth valve (505) provided in the gas exhaust pipe (550) to block or open a flow of gas exhausted to the outside through the gas exhaust pipe (550).

13. A gas collecting method using the gas collecting apparatus of claim 12, comprising:
a vacuum forming step of forming a vacuum inside the outer housing (200) and the dilution tank (520) through the vacuum pump unit (530);
a perforated hole forming step of forming the perforated hole in the battery by the punching unit (300);
a pressure change value calculating step of calculating a pressure change value through the measured value of the pressure sensor (570);
a needle retracting step of retracting the needle when the pressure change value is greater than or equal to a set value; and
a gas collecting step of opening the fourth valve and delivering gas to the gas collecting pipe (12) or the gas analyzer (13).

## Patentansprüche

1. Gassammelvorrichtung aufweisend:
eine innere Spannvorrichtung (100) mit einer Batterieaufnahmenut, in die eine Batterie eingesetzt ist, die auf einer oberen Oberfläche ausgebildet ist;
ein äußeres Gehäuse (200), das die innere Spannvorrichtung (100) darin aufnimmt, so dass eine äußere Umfangsoberfläche der inneren Spannvorrichtung (100) an ihrer inneren Umfangsoberfläche haftet;
ein Gasauslassrohr (221), das mit dem äußeren Gehäuse (200) verbunden ist und konfiguriert ist, um Gas, das von der Batterie erzeugt wird, an ein Gassammelrohr (12) oder einen Gasanalysator (13) zu liefern, das außerhalb des äußeren Gehäuses (200) vorgesehen ist;
eine Stanzeinheit (300), die an einem oberen Ende des äußeren Gehäuses (200) angeordnet ist und ein perforiertes Loch zur Gasabgabe auf einer oberen Oberfläche der Batterie bildet; und
eine Stanzantriebseinheit (400), die konfiguriert ist, um eine Antriebskraft zum Antreiben der Stanzeinheit (300) in einer vertikalen Richtung bereitzustellen,
wobei die Stanzeinheit (300) aufweist:
eine Stanznadel (310), die innerhalb des äußeren Gehäuses (200) durch ein Nadeleinsetzloch (211) eingesetzt ist, das an dem oberen Ende des äußeren Gehäuses (200) vorgesehen ist, wobei ein unteres Ende der Stanznadel (310) die obere Oberfläche der Batterie perforiert, um das perforierte Loch auf der oberen Oberfläche der Batterie zu bilden;
eine Stanzstange (320), die konfiguriert ist, um sich in der vertikalen Richtung durch die Stanzantriebseinheit (400) zu bewegen, wobei ein oberes Ende der Stanznadel (310) mit einem unteren Ende der Stanzstange (320) gekoppelt ist; und
eine Verschlussabdeckung (330), die konfiguriert ist, um eine äußere Umfangsoberfläche der Stanzstange (320) zu umgeben, wobei ein unteres Ende der Dichtungsabdeckung (330) mit dem oberen Ende des äußeren Gehäuses (200) gekoppelt ist,
wobei die Stanzantriebseinheit (400) aufweist:
ein Bewegungselement (410), an dem ein oberes Ende der Dichtungsabdeckung (330) und ein oberes Ende der Stanzstange (320) befestigt sind;
ein Stützelement (420), das an einer äußeren Umfangsoberfläche des äußeren Gehäuses (200) befestigt ist; und
ein Antriebsmittel (430), das an dem Stützelement (420) befestigt ist und konfiguriert ist, um das Bewegungselement (410) in der vertikalen Richtung zu bewegen.

2. Gassammelvorrichtung nach Anspruch 1, wobei die Verschlussabdeckung (330) als ein Balg bereitgestellt ist, der sich in der vertikalen Richtung ausdehnt oder zusammenzieht,
ein oberes Ende der Verschlussabdeckung (330) mit einem Bewegungselement (410) der Stanzantriebseinheit (400) zusammen mit einem oberen Ende der Stanzstange (320) gekoppelt ist, und
das untere Ende der Verschlussabdeckung (330) das Nadeleinsetzloch (211) abdeckt und mit dem oberen Ende des äußeren Gehäuses (200) gekoppelt ist.

3. Gassammelvorrichtung nach Anspruch 1, wobei das Antriebsmittel (430) aufweist:
eine Welle (431), die sich in der vertikalen Richtung erstreckt und mit einem Schraubengewinde an ihrer äußeren Umfangsoberfläche bereitgestellt ist; und
einen Motor (432), der konfiguriert ist, um die Welle (431) zu drehen, während sie durch das Stützelement (420) gestützt wird, und
wobei in dem Bewegungselement (410) ein Welleneinsetzloch (431), in das die Welle (431) eingesetzt ist, ausgebildet ist, und
an einer inneren Umfangsoberfläche des Welleneinsetzlochs (431) ein Schraubengewinde, das mit dem Schraubengewinde der Welle (431) in Eingriff steht, ausgebildet ist.

4. Gassammelvorrichtung nach Anspruch 1, wobei das äußere Gehäuse (200) aufweist:
ein oberes Gehäuse (210), in dem ein oberes Ende der inneren Spannvorrichtung (100) aufgenommen ist; und
ein unteres Gehäuse (220), in dem ein unteres Ende der inneren Spannvorrichtung (100) aufgenommen ist, und
wobei an einer oberen Oberfläche des oberen Gehäuses (210) das Nadeleinsetzloch (211) ausgebildet ist, und
die Stanzeinheit (300) mit der oberen Oberfläche des oberen Gehäuses (210) gekoppelt ist, während sie das Nadeleinsetzloch (211) von einer Außenseite des oberen Gehäuses (210) abdeckt.

5. Gassammelvorrichtung nach Anspruch 4, wobei die innere Spannvorrichtung (100) aufweist:
eine obere Spannvorrichtung (110), die mit einem Batterieeinsetzloch (111) versehen ist, in das ein oberes Ende der Batterie eingesetzt ist; und
eine untere Spannvorrichtung (120), die mit einer Batterieeinsetznut (121) versehen ist, in die ein unteres Ende der Batterie auf einer oberen Oberfläche eingesetzt ist, und
wobei das Batterieeinsetzloch (111) ausgebildet ist, um die obere Spannvorrichtung (110) in der vertikalen Richtung zu durchdringen,
eine obere Öffnung des Batterieeinsetzlochs (111) dem Nadeleinsetzloch (211) in einer Innenseite des oberen Gehäuses (210) zugewandt ist, und
eine untere Öffnung des Batterieeinsetzlochs (111) einem Einlass der Batterieeinsetznut (121) zugewandt ist.

6. Gassammelvorrichtung nach Anspruch 5, wobei das Gasauslassrohr (221) mit einer Seitenoberfläche des unteren Gehäuses (220) gekoppelt ist,
in der oberen Spannvorrichtung (110) ein erster Strömungsweg (115) ausgebildet ist, der konfiguriert ist, um Gas, das von einem Inneren der Batterie austritt und durch das perforierte Loch in das obere Gehäuse (210) diffundiert, an das Innere des unteren Gehäuses (220) zu liefern, und
in der unteren Spannvorrichtung (120) ein zweiter Strömungsweg (125) ausgebildet ist, der konfiguriert ist, um das Gas, das durch den ersten Strömungsweg (115) geliefert wird, an das Gasauslassrohr (221) zu liefern.

7. Gassammelvorrichtung nach Anspruch 6, wobei eine obere Oberfläche der oberen Spannvorrichtung (110) von einer Deckenoberfläche in einem vorbestimmten Intervall innerhalb des oberen Gehäuses (210) beabstandet ist,
die obere Spannvorrichtung (110) und die untere Spannvorrichtung (120) in einer Säulenform bereitgestellt sind, die sich in der vertikalen Richtung erstreckt,
der erste Strömungsweg (115) als eine Nut ausgebildet ist, die sich von einem oberen Ende zu einem unteren Ende der oberen Spannvorrichtung (110) auf einer äußeren Oberfläche der oberen Spannvorrichtung (110) erstreckt, und
der zweite Strömungsweg (125) als eine Nut ausgebildet ist, die sich von einem oberen Ende der unteren Spannvorrichtung (120) bis zu einer Höhe erstreckt, bei der das Gasauslassrohr (221) mit dem unteren Gehäuse (220) auf einer äußeren Oberfläche der unteren Spannvorrichtung (120) verbunden ist.

8. Gassammelvorrichtung nach Anspruch 7, wobei die obere Spannvorrichtung (110) und die untere Spannvorrichtung (120) in einer zylindrischen Form bereitgestellt sind, die sich in der vertikalen Richtung erstreckt,
an einem unteren Ende einer Seitenoberfläche der oberen Spannvorrichtung (110) oder dem oberen Ende der unteren Spannvorrichtung (120) ein erster Verbindungsströmungsweg (117) ausgebildet ist, der als eine ringförmige Nut entlang einer äußeren Umfangsoberfläche bereitgestellt ist, und
ein unteres Ende des ersten Strömungswegs (115) und ein oberes Ende des zweiten Strömungswegs (125) mit dem ersten Verbindungsströmungsweg (117) verbunden sind.

9. Gassammelvorrichtung nach Anspruch 8, wobei ein zweiter Verbindungsströmungsweg (127), der als eine ringförmige Nut entlang der äußeren Umfangsoberfläche bereitgestellt ist, in der unteren Spannvorrichtung (120) ausgebildet ist,
ein unteres Ende des zweiten Strömungswegs (125) mit dem zweiten Verbindungsströmungsweg (127) verbunden ist, und
eine Höhe des zweiten Verbindungsströmungswegs (127) die gleiche wie die Höhe ist, bei der das Gasauslassrohr (221) mit dem unteren Gehäuse (220) verbunden ist.

10. Gassammelvorrichtung nach Anspruch 1, ferner aufweisend:
einen Drucksensor (570), der konfiguriert ist, um einen Druck innerhalb des äußeren Gehäuses (200) zu messen,
wobei die Stanzantriebseinheit (400) basierend auf einem Messwert des Drucksensors (570) betrieben wird.

11. Gassammelvorrichtung nach Anspruch 10, ferner aufweisend:
eine Verteilereinheit (500), die mit dem Gasauslassrohr (221) verbunden ist, um das Gas, das von der Batterie erzeugt wird, von dem äußeren Gehäuse (200) aufzunehmen;
eine Verdünnungstankeinheit (520), die mit der Verteilereinheit (500) verbunden ist, um das Gas, das an die Verteilereinheit (500) geliefert wird, zu verdünnen;
eine Vakuumpumpeneinheit (530), die mit der Verteilereinheit (500) verbunden ist, um ein Vakuum in der Verdünnungstankeinheit (520) zu bilden; und
ein Gaslieferrohr (540), das mit der Verteilereinheit (500) verbunden ist, um das Gas an das Gassammelrohr (12) oder die Gassammelvorrichtung zu liefern,
wobei der Drucksensor (570) mit der Verteilereinheit (500) verbunden ist.

12. Gassammelvorrichtung nach Anspruch 11, aufweisend:
ein erstes Ventil (501), das in dem Gasauslassrohr (221) vorgesehen ist, um eine Strömung von Gas, das durch das Gasauslassrohr (221) zu der Verteilereinheit (500) strömt, zu blockieren oder zu öffnen;
ein zweites Ventil (502), das konfiguriert ist, um einen Strömungsweg zwischen der Verdünnungstankeinheit (520) und der Verteilereinheit (500) zu blockieren oder zu öffnen;
ein drittes Ventil (503), das konfiguriert ist, um einen Strömungsweg zwischen der Vakuumpumpeneinheit (530) und der Verteilereinheit (500) zu blockieren oder zu öffnen;
ein viertes Ventil (504), das in dem Gaslieferrohr (540) vorgesehen ist, um eine Strömung von Gas, das durch das Gaslieferrohr (540) zu dem Gassammelrohr (12) oder der Gassammelvorrichtung strömt, zu blockieren oder zu öffnen;
ein Gasaustrittsrohr (550), das mit der Verteilereinheit (500) verbunden ist, um das Gas, das an die Verteilereinheit (500) geliefert wird, nach außen abzuführen; und
ein fünftes Ventil (505), das in dem Gasaustrittsrohr (550) vorgesehen ist, um eine Strömung von Gas, das durch das Gasaustrittsrohr (550) nach außen abgeführt wird, zu blockieren oder zu öffnen.

13. Gassammelverfahren unter Verwendung der Gassammelvorrichtung nach Anspruch 12, aufweisend:
einen Vakuumbildungsschritt des Bildens eines Vakuums innerhalb des äußeren Gehäuses (200) und des Verdünnungstanks (520) durch die Vakuumpumpeneinheit (530);
einen Schritt des Bildens eines perforierten Lochs des Bildens des perforierten Lochs in der Batterie durch die Stanzeinheit (300);
einen Druckänderungswert-Berechnungsschritt des Berechnens eines Druckänderungswerts durch den gemessenen Wert des Drucksensors (570);
einen Nadelrückzugsschritt des Zurückziehens der Nadel, wenn der Druckänderungswert größer oder gleich einem eingestellten Wert ist; und
einen Gassammelschritt des Öffnens des vierten Ventils und des Lieferns von Gas an das Gassammelrohr (12) oder den Gasanalysator (13).

## Revendications

1. Appareil de collecte de gaz, comprenant :
un bâti intérieur (100) avec une rainure d'assise pour batterie dans laquelle une batterie est insérée, formée sur une surface supérieure ;
un boîtier extérieur (200) accueillant le bâti intérieur (100) de sorte qu'une surface circonférentielle extérieure du bâti intérieur (100) adhère à sa surface circonférentielle intérieure ;
un tuyau d'évacuation du gaz (221) relié au boîtier extérieur (200), et configuré pour apporter le gaz généré par la batterie à un tuyau collecteur de gaz (12) ou un analyseur de gaz (13) agencé hors du boîtier extérieur (200) ;
un dispositif de poinçonnage (300) situé sur une extrémité supérieure du boîtier extérieur (200) et formant un orifice perforé pour l'évacuation du gaz sur une surface supérieure de la batterie ; et
un module de commande de poinçonnage (400) configuré pour fournir une force motrice pour l'entraînement du dispositif de poinçonnage (300) dans le sens vertical,
le dispositif de poinçonnage (300) comprenant :
une aiguille de poinçonnage (310) insérée à l'intérieur du boîtier extérieur (200) par un trou d'insertion d'aiguille (211) agencé à l'extrémité supérieure du boîtier extérieur (200), une extrémité inférieure de l'aiguille de poinçonnage (310) perforant la surface supérieure de la batterie pour former l'orifice perforé sur la surface supérieure de la batterie ;
une tige de poinçonnage (320) configurée pour se déplacer dans le sens vertical par le module de commande de poinçonnage (400), une extrémité supérieure de l'aiguille de poinçonnage (310) étant couplée à une extrémité inférieure de la tige de poinçonnage (320) ; et
un couvercle d'étanchéité (330) configuré pour entourer une surface circonférentielle extérieure de la tige de poinçonnage (320), une extrémité inférieure du couvercle d'étanchéité (330) étant couplée à l'extrémité supérieure du boîtier extérieur (200),
le module de commande de poinçonnage (400) comprenant :
un élément mobile (410) auquel sont fixées une extrémité supérieure du couvercle d'étanchéité (330) et une extrémité supérieure de la tige de poinçonnage (320) ;
un élément de support (420) fixé à une surface circonférentielle extérieure du boîtier extérieur (200) ; et
un dispositif d'entraînement (430) fixé à l'élément de support (420) et configuré pour déplacer l'élément mobile (410) dans le sens vertical.

2. Appareil de collecte de gaz selon la revendication 1, le couvercle d'étanchéité (330) étant agencé sous forme d'un soufflet se déployant ou se contractant dans le sens vertical,
une extrémité supérieure du couvercle d'étanchéité (330) étant couplée à un élément mobile (410) du module de commande de poinçonnage (400), ainsi qu'à une extrémité supérieure de la tige de poinçonnage (320), et
l'extrémité inférieure du couvercle d'étanchéité (330) couvrant l'orifice d'insertion de l'aiguille (211), et étant couplée à l'extrémité supérieure du boîtier extérieur (200).

3. Appareil de collecte de gaz selon la revendication 1, le dispositif d'entraînement (430) comprenant :
un arbre (431) déployé dans le sens vertical, et doté d'un filetage sur sa surface circonférentielle extérieure ; et
un moteur (432) configuré pour entraîner l'arbre (431) tout en étant soutenu par l'élément de support (420), et
de sorte que, dans l'élément mobile (410), soit formé un orifice d'insertion de l'arbre (431) dans lequel l'arbre (431) est inséré, et
sur une surface circonférentielle intérieure de l'orifice d'insertion de l'arbre (431) étant formé un filetage s'engageant avec le filetage de l'arbre (431).

4. Appareil de collecte de gaz selon la revendication 1, le boîtier extérieur (200) comprenant :
un boîtier supérieur (210) accueillant une extrémité supérieure du bâti intérieur (100) ; et
un boîtier inférieur (220) accueillant une extrémité inférieure du bâti intérieur (100), et
sur une surface supérieure du boîtier supérieur (210) étant formé l'orifice d'insertion de l'aiguille (211), et
le dispositif de poinçonnage (300) étant couplé à la surface supérieure du boîtier supérieur (210) tout en couvrant l'orifice d'insertion de l'aiguille (211) depuis un côté extérieur du boîtier supérieur (210).

5. Appareil de collecte de gaz selon la revendication 4, le bâti intérieur (100) comprenant :
un bâti supérieur (110) muni d'un orifice d'insertion de la batterie (111) dans lequel une extrémité supérieure de la batterie est insérée ; et
un bâti inférieur (120) muni d'une rainure d'insertion de la batterie (121) dans lequel une extrémité inférieure de la batterie est insérée sur une surface supérieure ; et
l'orifice d'insertion de la batterie (111) étant formé pour pénétrer dans le bâti supérieur (110) dans le sens vertical,
une ouverture supérieure de l'orifice d'insertion de la batterie (111) faisant face à l'orifice d'insertion de l'aiguille (211) dans un côté intérieur du boîtier supérieur (210), et
une ouverture inférieure de l'orifice d'insertion de la batterie (111) faisant face à une entrée de la rainure d'insertion de la batterie (121).

6. Appareil de collecte de gaz selon la revendication 5, le tuyau de décharge du gaz (221) étant couplé à une surface latérale du boîtier inférieur (220),
dans le bâti supérieur (110) étant formé un premier chemin d'écoulement (115) configuré pour amener du gaz déchargé de l'intérieur de la batterie par l'orifice perforé et diffusé à l'intérieur du boîtier supérieur (210) à l'intérieur du boîtier inférieur (220), et
dans le bâti inférieur (120) étant formé un deuxième chemin d'écoulement (125) configuré pour amener le gaz distribué par le premier chemin d'écoulement (115) au tuyau d'évacuation du gaz (221).

7. Appareil de collecte de gaz selon la revendication 6, une surface supérieure du bâti supérieur (110) étant espacée d'une surface supérieure à un intervalle prédéterminé à l'intérieur du boîtier supérieur (210),
le bâti supérieur (110) et le bâti inférieur (120) étant agencés sous forme d'une colonne s'étendant dans le sens vertical,
le premier chemin d'écoulement (115) étant formé comme une rainure s'étendant d'une extrémité supérieure à une extrémité inférieure du bâti supérieur (110) sur une surface extérieure du bâti supérieur (110), et
le deuxième chemin d'écoulement (125) étant formé sous forme d'une rainure s'étendant d'une extrémité supérieure du bâti inférieur (120) jusqu'à une hauteur à laquelle le tuyau d'évacuation du gaz (221) est relié au boîtier inférieur (220) sur une surface extérieure du bâti inférieur (120).

8. Appareil de collecte de gaz selon la revendication 7, le bâti supérieur (110) et le bâti inférieur (120) étant agencés sous une forme cylindrique s'étendant dans le sens vertical,
à une extrémité inférieure d'une surface latérale du bâti supérieur (110) ou à l'extrémité supérieure du bâti inférieur (120), un premier chemin d'écoulement de connexion (117), agencé comme une rainure annulaire le long d'une surface circonférentielle extérieure, étant formé, et
une extrémité inférieure du premier chemin d'écoulement (115) et une extrémité supérieure du deuxième chemin d'écoulement (125) étant raccordés au premier chemin d'écoulement de connexion (117).

9. Appareil de collecte de gaz selon la revendication 8, un deuxième chemin d'écoulement de connexion (127) agencé comme une rainure annulaire le long de la surface circonférentielle extérieure étant formé dans le bâti inférieur (120),
une extrémité inférieure du deuxième chemin d'écoulement (125) étant raccordée au deuxième chemin d'écoulement de connexion (127), et
une hauteur du deuxième chemin d'écoulement de connexion (127) étant la même que celle à laquelle le tuyau d'évacuation du gaz (221) est raccordé au boîtier inférieur (220).

10. Appareil de collecte de gaz selon la revendication 1, comprenant en outre :
un capteur de pression (570) configuré pour mesurer une pression à l'intérieur du boîtier extérieur (200),
le module de commande de poinçonnage (400) étant actionné en fonction d'une valeur mesurée du capteur de pression (570).

11. Appareil de collecte de gaz selon la revendication 10, comprenant en outre :
un collecteur (500) relié au tuyau d'évacuation de gaz (221) pour recevoir du boîtier extérieur (200) le gaz généré par la batterie ;
un bac de dilution (520) relié au collecteur (500) pour diluer le gaz apporté au collecteur (500) ;
une unité de pompe à vide (530) reliée au collecteur (500) pour former un vide dans le bac de dilution (520) ; et
un tuyau de distribution de gaz (540) relié au collecteur (500) pour acheminer le gaz au tuyau collecteur de gaz (12) ou l'appareil de collecte de gaz,
le capteur de pression (570) étant connecté au collecteur (500).

12. Appareil de collecte de gaz selon la revendication 11, comprenant :
une première vanne (501) agencée dans le tuyau d'évacuation du gaz (221) pour bloquer ou ouvrir un débit de gaz s'écoulant dans le tuyau d'évacuation du gaz (221) jusqu'au collecteur (500) ;
une deuxième vanne (502) configurée pour bloquer ou ouvrir un chemin d'écoulement entre le bac de dilution (520) et le collecteur (500) ;
une troisième vanne (503) configurée pour bloquer ou ouvrir un chemin d'écoulement entre la pompe à vide (530) et le collecteur (500) ;
une quatrième vanne (504) agencée dans le tuyau de distribution de gaz (540) pour bloquer ou ouvrir un flux de gaz passant par le tuyau de distribution de gaz (540) jusqu'au tuyau collecteur de gaz (12) ou l'appareil de collecte de gaz ;
un tuyau de refoulement du gaz (550) relié au collecteur (500) pour évacuer à l'extérieur le gaz apporté au collecteur (500) ; et
une cinquième vanne (505) agencée dans le tuyau de refoulement du gaz (550) pour bloquer ou ouvrir un flux de gaz évacué vers l'extérieur à travers le tuyau de refoulement du gaz (550).

13. Procédé de collecte de gaz utilisant l'appareil de collecte de gaz selon la revendication 12, comprenant :
une étape de formation sous vide consistant à former un vide à l'intérieur du boîtier extérieur (200) et du bac de dilution (520) par le biais de la pompe à vide (530) ;
une étape de formation d'un orifice perforé consistant à former l'orifice perforé dans la batterie par le dispositif de poinçonnage (300) ;
une étape de calcul de la valeur de la variation de la pression comportant le calcul d'une valeur de variation de la pression à l'aide de la valeur mesurée du capteur de pression (570) ;
une étape de rétraction de l'aiguille consistant à rétracter l'aiguille lorsque la valeur de variation de pression est supérieure ou égale à une valeur fixe ; et
une étape de collecte de gaz consistant à ouvrir la quatrième vanne et à distribuer le gaz au tuyau collecteur de gaz (12) ou à l'analyseur de gaz (13).
